# EUROPEAN PATENT SPECIFICATION

(11) **EP 0 977 881 B1**
(45) Date of publication and mention of the grant of the patent: **02.03.2005**
(21) Application number: 98905562.9
(22) Date of filing: 13.03.1998
(51) Int. Cl.: C12N 15/86, C07K 14/15, C12N 7/01, C12N 5/10, A61K 35/76

(54) **EXPRESSION OF A MODIFIED FOAMY VIRUS ENVELOPE PROTEIN**
EXPRIMIERUNG EINES MODIFIZIERTEM "FOAMY VIRUS ENVELOPE PROTEIN" (HÜLLENPROTEIN)
EXPRESSION D'UNE PROTEINE D'ENVELOPPE MODIFIEE DE VIRUS SPUMEUX

(30) Priority: 13.03.1997 CA 2199989
(43) Date of publication of application: 09.02.2000
(73) Proprietor: TRANSGENE S.A., 67082 Strasbourg Cédex (FR)
(72) Inventor: RETHWILM, Axel, D-97078 Würzburg (DE); LINDEMANN, Dirk, D-97222 Rimpar (DE); WINTER, Arend, Jan, F-67100 Strasbourg (FR)
(74) Representative: Warcoin, Jacques
(86) International application number: PCT/IB1998/000343
(87) International publication number: WO 1998/040507

(56) References cited:
- MAHNKE C ET AL: "SPECIFIC ELISA FOR THE DETECTION OF ANTIBODIES TO THE HUMAN SPUMAVIRUS." J VIROL METHODS 29 (1). 1990. 13-22. CODEN: JVMEDH ISSN: 0166-0934, XP002070567
- GOEPFERT P A ET AL: "A SORTING MOTIF LOCALIZES THE FOAMY VIRUS GLYCOPROTEIN TO THE ENDOPLASMIC RETICULUM" JOURNAL OF VIROLOGY, vol. 71, no. 1, January 1997, pages 778-784, XP000674743
- LINDEMANN D ET AL: "Efficient pseudotyping of murine leukemia virus particles with chimeric human foamy virus envelope proteins." JOURNAL OF VIROLOGY. ISSN: 0022-538X, vol. 71, no. 6, June 1997, pages 4815-4820, XP002070568

## Description

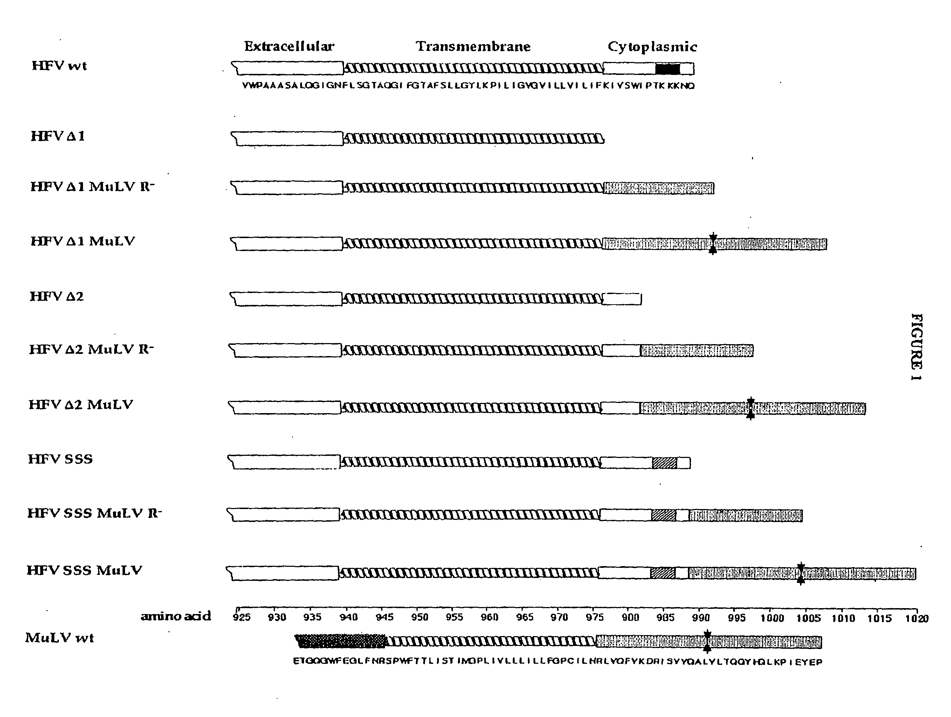

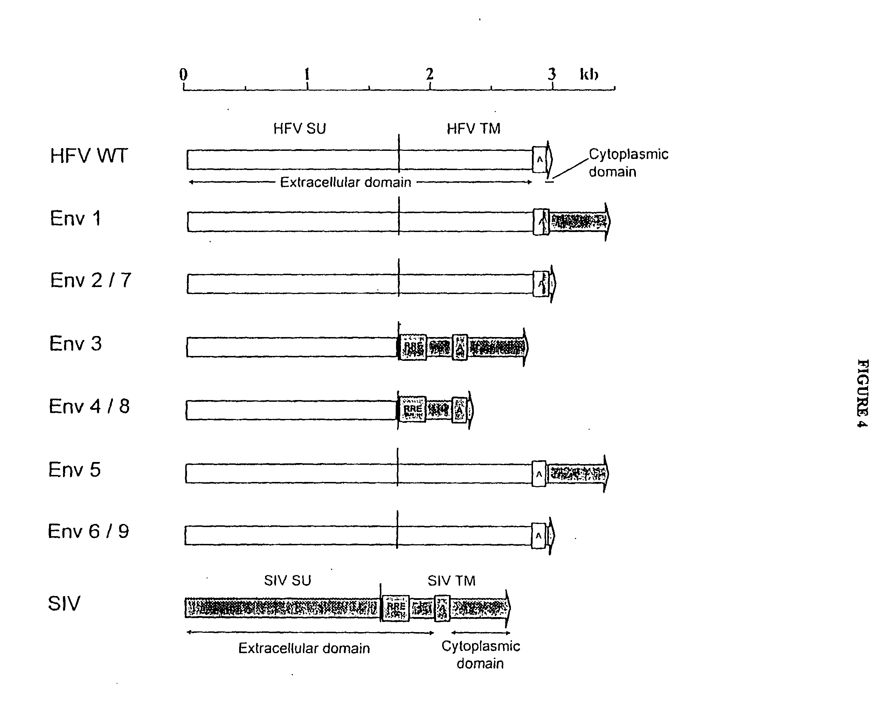

The foamy viruses (FV) subgroup of retroid viruses has attracted scientific interest because of their unique replication strategy and because of their potential use as gene transfer vectors (35). It has been proposed that FVs may be ideal tools for the development of a gene delivery system due to specific properties of this virus group, such as the absence of FV antibodies in the human population, the benign course of natural FV infections, their very broad host cell range, and an extended packaging limit due to the size of the FV genome (4, 30, 32). However, limited knowledge of the molecular biology of this virus group has so far not allowed the development of safe packaging cell lines and vectors, such as those that have been derived for murine retroviruses, among others (27). For instance, the FV is a DNA virus with a complex genome organization. In addition to LTRs (Long Terminal Repeat), a packaging region and gag, pol, env genes, it also comprises several genes such as bel1, bel2, bel3, bet, beo and bes located between env and 3'LTR. The env gene encodes a 130 kDa glycosylated precursor that is cleaved giving rise to the surface (SU) and transmembrane (TM) subunits (see Fig. 1 and 4). The TM subunit includes in its 3' part a transmembrane anchor domain (A in Fig 4) composed of hydrophobic residues which is followed by a cytoplasmic tail. Furthermore, FVs express their Pol protein from a spliced mRNA independently of the Gag protein, and the mechanism of FV genome packaging and particle assembly, as well as the significance of high amounts of reverse transcribed DNA in the extra-cellular particle are largely unknown (10, 18, 39). Other unique features include the nuclear localization of the Gag precursor protein (31, 40) and the predominant budding into intracytoplasmic vesicles which may be a consequence of the retention of the Env precursor protein in the ER (13).

Moloney retrovirus-based gene transfer vectors are currently the main vehicles for high efficiency stable gene transfer into a wide variety of cell types (20). Major limitations of this vector system are the restricted host cell range and the inefficient infectivity for some human cells (reviewed in (1)). Recently, several methods using the pseudotyping with foreign envelope proteins, such as the vesicular stomatitis virus (VSV) G glycoprotein (6, 38) or the gibbon ape leukemia virus (GALV) envelope protein (2, 34) have been shown to overcome these disadvantages.

However, the expression of VSV-G for example is highly toxic for the producer cells and has prevented the generation of stable VSV-G packaging cells line (8, 22, 37).

Some modifications in the *env* protein of the foamy virus have already been disclosed. In fact, Mahnke et al. (J. Virol. Methods, 29(1), 1990, 13-22) disclose constructs for the expression of HSRV (Human Foamy Virus) *env* protein fused to MS2 polymerase peptide, and the resulting expression of said fusion protein in *E. coli,* and Goepfert et al. (Journal of Virology, vol. 71, n°1, 1997, 778-784) disclose constructs with altered HFV *env* codons.

However, the invention concerns constructs for the expression of a protein comprising at least a modified FV envelope protein which is truncated at aa 975 or, more preferably, 981. The truncation may extend up to the stop codon or alternatively comprise before the stop codon one or several residues optionally from the original FV env protein.

The modified FV env protein in use in the present invention is a fusion protein which furthermore comprises all or preferably a part of a non-FV envelope protein.

The preferred FV according to the present invention is the human foamy virus (HFV), but others may be used (e.g. Simian FV)

Examples of suitable non-FV viruses include avian retroviruses, bovine retroviruses, feline retroviruses, murine retroviruses such as Murine Leukemia Virus (MuLV) and particularly Moloney MuLV (MoMuLV), Friend Murine Leukemia Virus (FrMuLV) especially strain FB 29, Murine Sarcome Virus (MSV), primate retroviruses such as GaLV, VSV or lentiviruses such as HIV (Human Immunodeficiency Virus) or SIV (Simian Immunodeficiency Virus).

The fusion betwen FV and non-FV env proteins can be made at different locations. Fusions within the TM subunit are advantageous. According to a first alternative, the fusion is within the transmembrane anchor domain of said FV and non-FV envelope proteins. A prefered example is a protein that comprises the extracellular domain and the 5' part of the transmembrane anchor domain of the HFV envelope protein and the 3' part of the transmembrane anchor domain and the cytoplasmic domain of the non-FV envelope protein, particularly of the SIV envelope protein.

A second alternative is that the fusion is within the cleavage site of said FV and non-FV envelope proteins. A prefered example is a protein that comprises the SU domain and all or part of the cleavage site of the HFV envelope protein and all or part of the cleavage site and the TM domain, comprising the transmembrane anchor domain and the cytoplasmic domain, of the non-FV envelope protein, particularly of the SIV envelope protein. The replacement of the cleavage site of the FV envelope protein by its equivalent from the non-FV envelope protein is also envisageable.

Another alternative is that the fusion is at the junction between the transmembrane anchor domain and the cytoplasmic domain or within the cytoplasmic domains of said FV and non-FV envelope proteins. A prefered example is a protein that comprises the extracellular domain, the transmembrane anchor domain and all or part of the cytoplasmic domain of the HFV envelope protein and all or part of the cytoplasmic domain of the non-FV envelope protein, particularly of the SIV envelope protein.

In a particularly preferred embodiment, a protein according to the invention consists in HFV protein envelope which all or part of the cytoplasmic domain is replaced by all or part of a cytoplasmic domain of a non-FV viral envelope protein, especially of a MuLV envelope protein. Advantageously, the fusion protein consists in the fusion of a MuLV cytoplasmic domain to a modified HFV envelope protein. The MuLV cytoplasmic domain in use in the present invention may be processed or unprocessed. «Processed» means that it contains the cleavage site normally recognized by the corresponding retroviral protease and «unprocessed» that it does not contain it or that it is not functional (mutation, deletion or truncation).

The preferred construct of the invention is the one allowing expression of the fusion protein designated hereinafter HFV Δ2 MuLV.

Alternatively, a prefered protein according to the invention consists in a HFV envelope protein in which all or part of the cytoplasmic tail is replaced by all or part of a cytoplasmic domain of a SIV envelope protein. There exists two versions of the SIV envelope protein : a long form having a cytoplasmic tail of 164 amino acids which is present in SIV particles replicating in the natural host, the rhesus monkey (Macaca mulatta) and a short form containing only 18 amino acids. This short form is selected for when virus isolated from the monkey are cultured on human cell lines (42).

It is also possible that the construct of the invention is mutated in the donor and/or acceptor splicing sites naturally present in the FV env protein encoding sequence.

The construct of the invention may include regulatory elements to allow transcription and translation of the sequence coding for the modified FV env protein. In particular, a suitable promoter may be linked upstream from the FV env encoding sequence in an operative way by conventional recombinant techniques. Such a promoter may be of prokaryote, eukaryote or viral origin and may be constitutive or regulated. Such regulatory elements are well known in the art.

It is also in the scope of the invention that the construct of the invention may additionally comprise a selection gene enabling detection and isolation of the cells expressing the modified FV env protein. In the context of the invention, the selection gene may be under the transcriptional control of the promoter driving expression of the modified FV env protein resulting in a bicistronic transcript or under the control of an additional promoter region. The possible selection genes are numerous, for example neo gene conferring resistance to antibiotic G418, dihydrofolate reductase (dhFr) gene, puromycin acetyl transferase (pac) gene or xanthine phosphoribosyl transferase (gpt).

The construct of the invention may be inserted in any appropriate vector, a viral vector (e.g. a retroviral vector) or a plasmid. The choice of the appropriate vector is large and within the capabilities of the man skilled in the art. Such a vector may be integrative or not. To decrease the possibility to generate replication-competent viral particles, it is advantageous that the construct lack any retroviral LTR and packaging region.

The invention also concerns fusion proteins as expressed by the above expression constructs as well as pseudotyped viral particles comprising a FV env protein. This latter may be derived from a native FV env protein, a part thereof or a modified one. In a preferred embodiment, the pseudotyped viral particle at its surface comprises a modified FV env protein as expressed by a construct according to the invention. The pseudotyped viral particle of the invention may be generated upon transfection of a recombinant viral vector into a complementation cell line. The technology is conventional and described in numerous prior art documents. A viral vector in use in the present invention comprises preferably at least a 5' LTR, a packaging region and a 3' LTR derived from any retrovirus such those cited previously and a gene capable of expressing a ribozyme, an anti-sense RNA molecule or a mRNA to further produce a polypeptide of interest. Of particular interest, are therapeutic polypeptides, including but not limited to cytokines (IL-2, IFN α, β or γ), Herpes Simplex Virus type 1 (HSV-1) thymidine kinase (TK), Cystic Fibrosis Transmembrane Conductance Regulator (CFTR), Dystrophin, coagulation Factors (FVIII, FIX, ...), tumor-associated antigens (MUC-1, HPV antigens), antibodies, immunotoxines, anti-HIV drugs, growth factors (Fibroblast Growth Factor FGF, Vascular Endothelial Growth Factor VEGF), apoptosis inducers (Bax...), apoptosis inhibitors (Bcl2, Bclx...), cytostatic agents (p21, p16, Rb), epo lipoproteins, nitrix oxid synthetase (Nos), oxigen radical scaveyers (SOD, catalase...), tumor suppressor products (p53, p73) and markers. This list is not limitative. Furthermore, the viral vector in use in the present invention may transfer one or more genes in a native, truncated, mutated or hybrid form. The gene(s) is placed under the control of elements allowing its expression in an eukaryotic cell. Such elements includes a promoter, which may be from any origin (retroviral LTR or internal promoter). It may be constitutive or responsive to cell or tissue-specific factors.

Another object of the invention is relating to complementation cell line permitting the production of the pseudotyped viral particles and the method of their preparation.

The invention further concerns complementation cell line comprising a construct of the invention.

Preferably, said complementation cell line comprises a construct of the invention characterized in that fusion is at junction between the transmembrane anchor domain and the cytoplasmic domain or within the cytoplamic domains of said FV and non-FV envelope proteins and in that the expressed protein comprises the extracellular domain, the transmembrane anchor domain and all or part of the cytoplasmic domain of the HFV envelope protein and all or part of the cytoplasmic domain of the non-FV envelope protein, particularly of the SIV envelope protein.

In another preferred embodiment, the said complementation cell line comprises a construct of the invention for the expression of a protein characterized in that the protein is HFVΔ2 MuLV.

The complementation cell line of the invention may derive from any cell and, particularly, eukaryotic cell. One may envisage murine cell lines, pharmaceutically acceptable cell lines (Vero, CHO, ...) or human cell line such as 293 or A549. It may be generated by transfection of a construct according to the invention along with a first selection gene. The highest env producer cells are then screened for expression of high levels of FV env protein by immunodetection using antibodies against FV env, Western blot, FACS (Fluorescente Activated Cell Sorter) or any other method. Alternatively the complementation cell line of the invention, may also comprise a construct expressing a viral gag / pol gene, more preferably of MuLV, FB 29, SIV or HFV along with a second selection gene different from the first one. Preferably, the env and gag / pol genes are carried by separate expression vector lacking LTR and packaging region. The selection and screening steps are repeated to select a env producing clone which further expresses gag / pol expression product.

A complementation cell line of the invention may be used to package recombinant viral vector. The titer may be tested using a conventional viral vector expressing a third selection gene different from the previous ones or a marker gene (e.g. Lac Z). As a result, cells producing high titers of pseudotyped viral particles are selected and can be cultured to supply a stable complementation cell line. The cells may also be tested transiently as usually performed and described hereinafter.

According to another aspect of the invention, it is also provided a method for preparing a pseudotyped viral particle of the invention. Such a method comprises the act of (1) introducing a recombinant retroviral vector into a complementation cell line of the invention, (2) culturing said complementation cell line under suitable conditions permitting production of the said pseudotyped viral particle and (3) recovering the resulting pseudotyped viral particle from cell culture.

Preferably, the pseudotyped viral particle is recovered from cell culture supernatant but a cell lysis step may also be considered. The pseudotyped viral particle may also be further purified by conventional technology (e.g. ultracentrifugation on sucrose or CICs gradient). Advantageously, the pseudotyped viral particle thus produced is able to infect (preferably in the absence of polycation such as polybrene) a wide variety of cells and optionally to resist to inactivation by human serum.

According to another aspect of the invention, it is also provided a mammalian host cell infected by the pseudotyped viral particle of the invention or obtainable by a method of the invention. Such a host cell includes without limitation human epithelial, pulmonary, muscular, hepatic, haematopoietic cells, fibroblastes and lymphocytes.

A pseudotyped infectious particle as well as a mammalian cell of the invention may be applied in the prevention or treatment of various diseases, as a vaccine or a therapeutic agent.

It is also the scope of the invention to provide for a pharmaceutical composition comprising a therapeutically or prophylactically effective amount of a pseudotyped viral particle of the invention or obtainable by a method of the invention as well as a mammalian cell of the invention as a therapeutic agent. Such a pharmaceutical composition may be produced in a conventional manner. In particular, the particle or the mammalian cell of the invention may be combined with appropriate substances well known in the art, such as a carrier, diluent, adjuvant or excipient. The particular formulation of the pharmaceutical composition depends on various parameters, for example the polypeptide of interest to be expressed, the desired site of action, the method of administration and the subject to be treated. Such a formulation can be determined by those skilled in the art and by conventional knowledge.

Typically, the pseudotyped particles are prepared as a solution in an acceptable diluent, such as saline, phosphate-buffered saline or other pharmaceutically acceptable diluent. The route of inoculation may be intraveinous, intramuscular, subcutaneous, intradermal, intrapulmonar, intratracheal, intragastric and intratumoral. The dose may be unique or repeated. The quantity will vary depending upon individuals (weight, sex, age, medical conditions....) and the disease to treat. In general, it is desirable to provide the pseudotyped viral particles of the invention in the range of from about 10⁴ to about 10¹³ plaque forming units (pfu)/dose, avantageously from about 10⁵ to about 10¹⁰ and preferably from about 10⁶ to about 10⁹. The composition of the invention can be introduced into a mammal (human) *ex vivo* by prealable exposure of target cells to the pseudotyped viral particles before introduction of the transduced cells into a mammal, or injected *in vivo* into the affected tissue, in the circulation or locally.

In a last embodiment of the invention, it is also provided a method of treating a genetic disorder or a disease induced by any pathogenic gene, such as cancer or a virally-induced disease, which comprises administering a therapeutically effective amount of a pseudotyped viral particle or a mammalian cell of the invention to a subject in need of a treatment.

These and other advantages of the subject invention will be apparent from the following examples and attached drawings. These embodiments do not represent the full scope of the invention.

In particular, incorporation of human foamy virus (HFV) envelope proteins into murine leukemia virus (MuLV) particles was studied in a transient transfection packaging cell system. We report here that wildtype HFV envelope protein can pseudotype MuLV particles, albeit at low efficiency. Complete or partial removal of the HFV cytoplasmic tail resulted in an abolishment or reduction of HFV mediated infectivity, implicating a role of the HFV envelope cytoplasmic tail in the pseudotyping of MuLV particles. Mutation of the ER retention signal present in the HFV envelope cytoplasmic tail did not result in a higher relative infectivity of pseudotyped retroviral vectors. However, a chimeric envelope protein, containing an unprocessed MuLV envelope cytoplasmic domain fused to a truncated HFV envelope protein, showed an enhanced HFV specific infectivity as a result of an increased incorporation of chimeric envelope proteins into MuLV particles.

### Brief description of the drawings

### Figure 1

### Schematic illustration of the HFV envelope expression constructs.

The extracellular, membrane spanning (also designated transmembrane anchor domain A), and cytoplasmic domains of the TM components of the HFV (open boxes) and the MuLV (shaded boxes) envelopes are shown according to (11, 24). The amino acid sequence of the wildtype HFV and MuLV proteins are given below the schematic illustration. The amino acid positions in the HFV envelope constructs are marked on the ruler. The location of the sequence motif in the cytoplasmic domain of the HFV envelope, responsible for ER retention (13) is indicated as a black box, the mutated sequence as a striped box. The cleavage site of the MuLV protease in the full length cytoplasmic domain of the MuLV envelope protein is indicated by two inverted arrows.

### Figure 2

### Infectivity of MuLV particles pseudotyped with different envelope proteins.

NIH3T3 (shaded bar) or QT-6 (solid bar) cells were infected with different pseudotyped MuLV particles generated by transient transfection of 293T cells. Forty-eight hours after transduction the percentage of GFP expressing cells was quantitated by FACS analysis. The mean fluorescence of GFP expressing cells was 100 to 300 fold above those of mock infected cells. The individual envelope constructs used for pseudotyping are indicated on the y-axis of the graph. The mean percentage of GFP expressing cells for each construct is shown on the x-axis with the corresponding standard deviation. Individual constructs were tested 2-6 times.

### Figure 3

### Neutralization of HFV envelope specific infectivity.

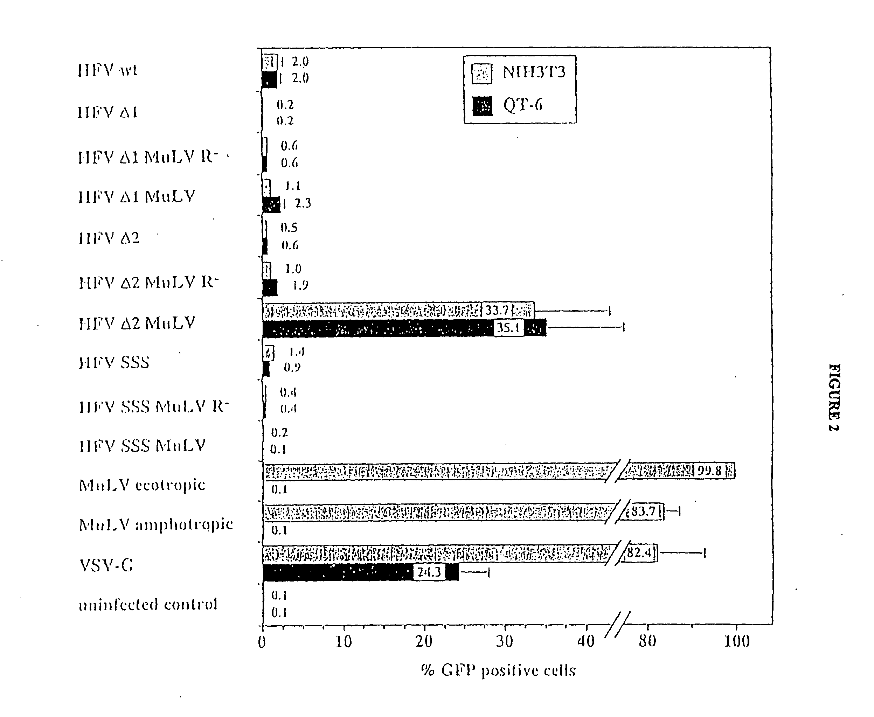

MuLV particles pseudotyped with different envelope proteins, as indicated on the y-axis, were generated by transient transfection of 293T cells. Supernatants (1 ml) were incubated with anti-HFV specific chimpanzee serum (1:60) (solid bar) or human serum (1:60) from a healthy individual (shaded bar) for 1 hour at 37°C, prior to the addition to NIH3T3 (A) or QT-6 (B) cells. The supernatant was aspirated four hours later, and replaced with fresh growth medium. Forty eight hours after transduction, the percentage of GFP expressing cells was determined as described in the legend of Fig. 2. The experiment was carried out twice with a neutralizing monkey serum and in addition, with an anti-HFV surface rabbit serum (data not shown) resulting in a similar relative inhibition of the infectivity of HFV envelope pseudotyped retroviral vectors.

### Figure 4

### Schematic representation of the chimeric HFV/SIV envelope constructs.

HFV WT and SIV are wild type versions of the human foamy virus and simian immunodeficiency virus (molecular clone mm251) envelope genes. Domains from HFV are in white and domains from SIV in grey. Cleavage site separing SU and TM domain is indicated with a vertical line. RRE represents rev responsive element and A the transmembrane anchor domain. The extracellular domain in 5' of the transmembrane anchor domain and the cytoplasmic domain in 3' of the transmembrane anchor domain are also indicated. Env 1 to 9 are representation of the chimeric HFV/SIV env constructs comprising fusion with long version (Env 1, 3 and 5) or short version (Env 2, 4, 6, 7, 8 and 9) of SIV envelope.

### Figure 5

### Sequence of the HFV, SIV and chimeric env genes flanking the fusion sites and amino acid sequences of the long and short cytoplasmic tails from SIV env.

**A.** illustrates the transmembrane anchor domains of HFV and SIV mm251 env genes and the fusion in the transmembrane anchor domain of HFV and SIV mm251 (Chim) : identical amino acids in the HFV and SIV envelopes are in bold, the transmembrane anchor domain sequences of HFV and SIV envelopes are underlined. **B**. illustrates the fusion at the cleavage site between SU and TM domains of HFV and SIV mm251 envelopes : consensus sequence of the cleavage site is in bold, the cleavage site is indicated by a space in the amino acid sequence. **C**. illustrates the substitution of the cytoplasmic tail of HFV envelope with the cytoplasmic tail of SIV mm251 envelope : the transmembrane anchor sequences are underlined, (141) indicates the last 141 amino acids from the SIV long cytoplasmic tail. **D**. amino acid sequences of the long and short cytoplasmic tails from SIV envelope : transmembrane anchor domains are underlined, * indicates a stop codon, (141) indicates the last 141 amino acids from the SIV long cytoplasmic tail.

### Figure 6

### Destruction of the SIV mm 251 env internal 3' splice site.

The amino acid sequence shown on top is from the SIVmm251 env gene, * indicates the stop codon of the humanized env gene. A indicates the G to A mutation resulting in the destruction of 3' splice site. Tat/Rev exon 2 starts at the sequence TAGACT but the reading frame is different from the env reading frame.

The present invention will now be illustrated in the following and non limitating examples.

### EXAMPLES

All constructions are made by using standard recombinant DNA techniques such as those described in T. Maniatis et al., Molecular cloning : a laboratory manual, Cold Spring Harbor, NY 1982. The cell lines are accessible by the culture collections such as ATCC and cultured by standard conditions (NIH3T3 : CRL-1658, Mv.1.Lu CCL64, HT 1080 CCL 121, BHK 21 CCL 10, QT 26 CRL 1708 and 293 CRL 1573). The sequence of the HFV env protein has already been published and is available in EMBL data base (accession number 407725).

### EXAMPLE 1: HFV/MLV chimeras.

### 1. Generation of FV env expression construct

An eukaryotic expression construct for the envelope gene of the human FV isolate (HFV) was generated by inserting a 3076 bp AflII/EcoRI fragment of the HFV proviral clone pHSRVl (28), containing the full-length env open reading frame (ORF), into the pCDNA3 (Invitrogen) vector. This construct was designated pCHFV wt and used to generate the mutant and chimeric HFV envelope proteins depicted in Fig. 1. Briefly, truncated or chimeric env constructs were made by using the polymerase chain reaction on HFV and/or MuLV env genes as templates and oligonucleotides incorporating the desired mutations. The mutants were inserted into the basic vector described above and sequenced to exclude off-site mutations. Three mutant HFV envelope constructs were generated. pCHFV Δl and pCHFV Δ2 code for HFV envelope proteins truncated at aa 975 or 981, respectively. pCHFV Δ2 has a C-terminal Arginine added, not present in the original HFV env sequence. According to the HFV envelope domain structure proposed by Flugel et al. (11) the truncations resulted in a complete (pCHFV Δl) or partial removal (pCHFV Δ2) of the cytoplasmic domain. Finally, the pCHFV SSS construct produces an HFV envelope protein that has the triple lysine motif (aa 984-986) at the C-terminal end of the cytoplasmic tail of the transmembrane (TM) protein replaced by serine residues. This sequence motif has been shown to be responsible for the ER retention of the HFV envelope (13, 14).

In total 6 chimeric envelope proteins were constructed by C-terminal fusion of sequences coding for the unprocessed or processed cytoplasmic domain of the MuLV envelope protein (16, 17). pCHFV ΔlMuLVR-, pCHFV Δ2MuLVR- and pCHFV SSSMuLVR- encode fusion proteins consisting of the 3 mutations described above and a processed MuLV envelope cytoplasmic domain (aa 634-649), whereas pCHFV ΔlMuLV, pCHFV Δ2MuLV and pCHFV SSSMuLV encode the respective fusion proteins containing an unprocessed MuLV envelope cytoplasmic domain (aa 634-665) at the C-terminus.

The expression constructs for the MuLV gag/pol (pHIT60), the ecotropic (pHITl23) and amphotropic (pHIT456) MuLV envelope were kindly provided by A. Kingsman (33). The retroviral vector SFG GFPS65T contains the humanized ORF of the green fluorescent protein (7) (a gift of M. Vogel) inserted into the cloning sites of the MuLV based retroviral vector SFG (5, 22), whereas MFG.S NLS-LacZ (22) contains the β-galactosidase gene fused to the SV40 nuclear localization signal (NLS) (a gift ofR. Mulligan). The VSV-G expression construct was generated by inserting a 1.6 kb EcoRI fragment from plasmid pSVGL-1 (29) (a gift of J. Rose) containing the VSV-G ORF, into the pHIT vector.

### 2. Infectivity of MuLV particles pseudotyped with various HFV env proteins

Recombinant retroviral particles were generated using the pHIT packaging system essentially as described previously (33). Briefly, 293T cells (9) were transiently co-transfected with an expression construct for MuLV gag / pol (pHIT60), the MuLV based retroviral vector SFG GFPS65T, and the different envelope expression constructs described above. Viral supernatants were harvested 48-72 hours after transfection. Supernatants from independent transfections with the same plasmids were pooled, filtrated (0.45 µm pore size), polybrene was added to a final concentration of 8 µg/ml, and the supernatants were used immediately or stored at -80°C until use. Target cells expressing the GFP protein after retroviral transduction were identified by FACS analysis on a FACScan, and the number of positive cells were quantitated using the LysisII and CellQuest Software package (Becton Dickinson).

Initial experiments using the pCHFV wt expression construct showed that MuLV particles can be pseudotyped with the HFV wt envelope protein and are able to transduce NIH3T3 cells, albeit at low efficiency (Fig. 2). The HFV envelope protein contains a signal sequence in its cytoplasmic domain that leads to a retention in the ER of expressing cells (13, 14). Therefore, three constructs, pCHFV Δl, pCHFV Δ2, and pCHFV SSS, coding for cytoplasmically truncated or mutated HFV envelope proteins were examined to determine the influence of the cytoplasmic domain of the HFV envelope and its ER retention on the pseudotyping efficiency. The complete (pCHFV Δl) or partial removal (pCHFV Δ2) of the cytoplasmic domain of the HFV envelope results in an abolishment or reduction of the already low pseudotyping activity observed for the wildtype protein (Fig. 2). Mutation of the cytoplasmic ER retention signal (pCHFV SSS) has previously been shown to increase cell surface expression of the HFV envelope protein (13). However, pseudotyping of viral particles with such a mutant protein also did not result in higher infectivity of these viruses (Fig. 2).

Since removal or modification of the HFV cytoplasmic domain failed to increase the infection efficiency of pseudotyped virus, a second approach has subsequently been used to test whether the replacement of the HFV cytoplasmic domain by the MuLV cytoplasmic domain, or the fusion of the MuLV cytoplasmic domain to a modified full-length HFV envelope would have the desired effect. The cytoplasmic domain of the MuLV envelope was shown to be processed by the MuLV protease in the viral particle (16, 17). Expression of an already processed form of the MuLV envelope protein in cells resulted in the formation of large multinucleated syncytia and a decrease of viral infectivity (24, 26). Therefore, C-terminal fusion proteins of the three mutants described above and the processed (MuLVR-) or the unprocessed (MuLV) cytoplasmic domain of the MuLV envelope protein were generated and particles pseudotyped with these chimeric envelope proteins were tested for their infectivity on NIH3T3 cells. Interestingly, viruses pseudotyped with one mutant, the HFV Δ2MuLV protein, showed a 10-20 fold higher infectivity than particles pseudotyped with the wildtype HFV envelope protein (Fig. 2). This increase in infectivity through the HFV Δ2MuLV protein was not specific for NIH3T3 or murine cells, as similar results were obtained for the quail fibroblast cell line QT-6, which is not infectable by viral particles coated with MuLV envelope proteins (Fig. 2). In these cells the infectivity of particles pseudotyped with the HFV Δ2MuLV envelope protein was consistently higher than those pseudotyped with the VSV-G protein. All other proteins analyzed gave rise to pseudotyped viruses with lower or similar relative infectivity when compared to wildtype HFV envelope on both cell lines (Fig. 2). In addition, chimeric HFV envelope proteins containing a processed MuLV cytoplasmic domain showed a higher fusion activity than the corresponding proteins having an unprocessed MuLV cytoplasmic domain upon expression in L929 cells by retroviral vectors (data not shown). This result is in accordance with data showing that the cytoplasmic domain of the MuLV envelope can control the fusion activity of foreign envelope proteins, such as the simian immunodeficiency virus (SIV), when expressed as a chimeric envelope protein (36). Furthermore, supernatants containing a retroviral vector coding for a nuclear localized β-galactosidase protein pseudotyped with the different envelope proteins were titrated on cell lines of various species (Table). More precisely, target cells (1 x 10⁴ cells/well) were plated 24 hours prior to infection with serial dilutions of supernatants of transfected 293T cells. Fourtyeight hours after infection the numbers of blue foci were counted in duplicates and the titers calculated. The values of the duplicates were within a 3-fold range. The results shown are a representative of two independent titrations on the cell lines indicated using for all cell lines cell free supernatants from the same transfections, with reproducible relative titers in both experiments. Supernatants containing pseudotyped particles were titrated up to 6 times on NIH3T3 cells with reproducible results. Retroviral particles pseudotyped with the HFV wt envelope protein or the HFV Δ2MuLV chimera were able to infect cells of human, mink quail and hamster origin, in addition to murine cells (Table 1). The titers of retroviral vectors pseudotyped with the HFV Δ2MuLV envelope protein were 8-35 fold higher than those pseudotyped with the wildtype HFV envelope protein depending on the target cells used.

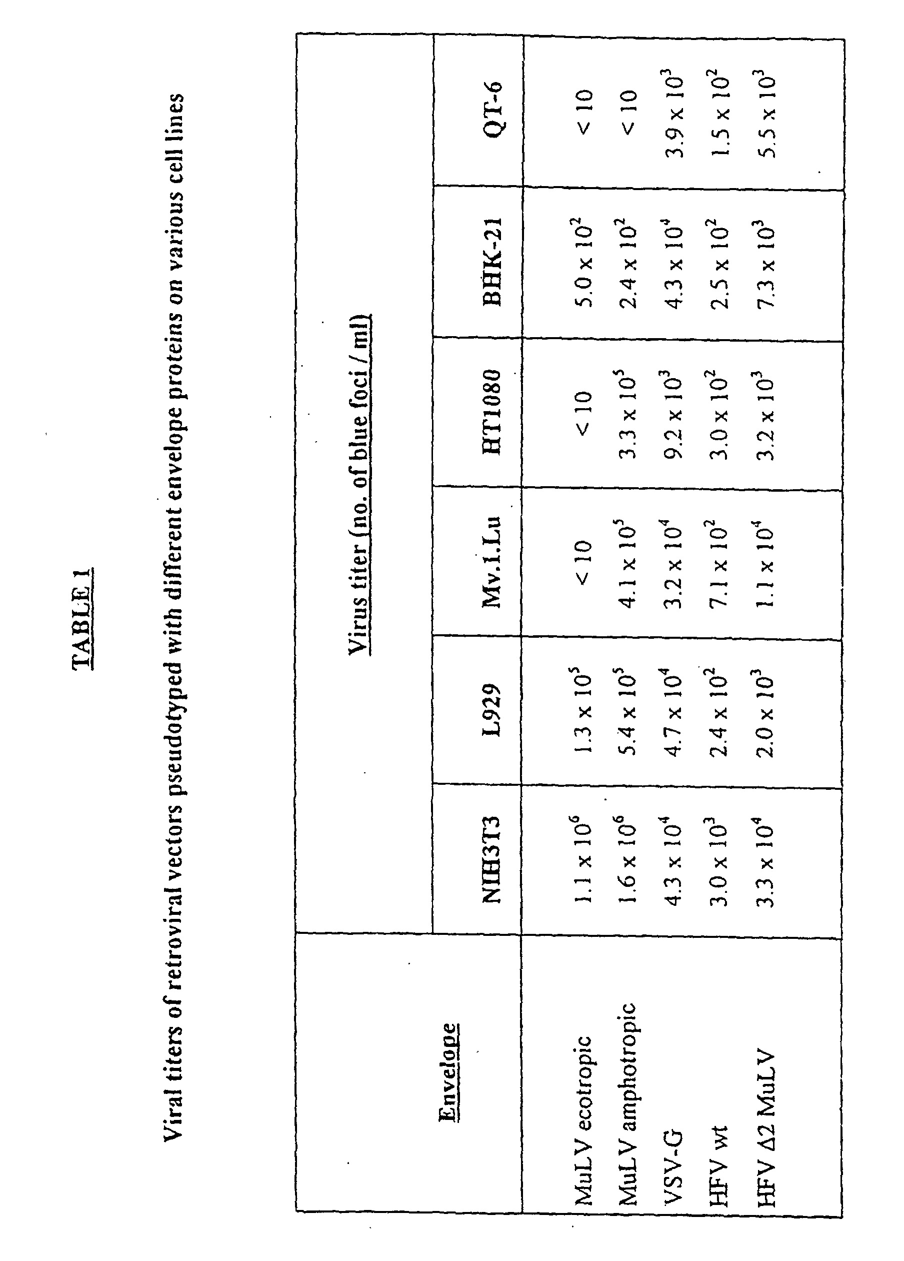

### 3. Neutralization of the infectivity of HFV env pseudotyped particles by HFV specific antisera

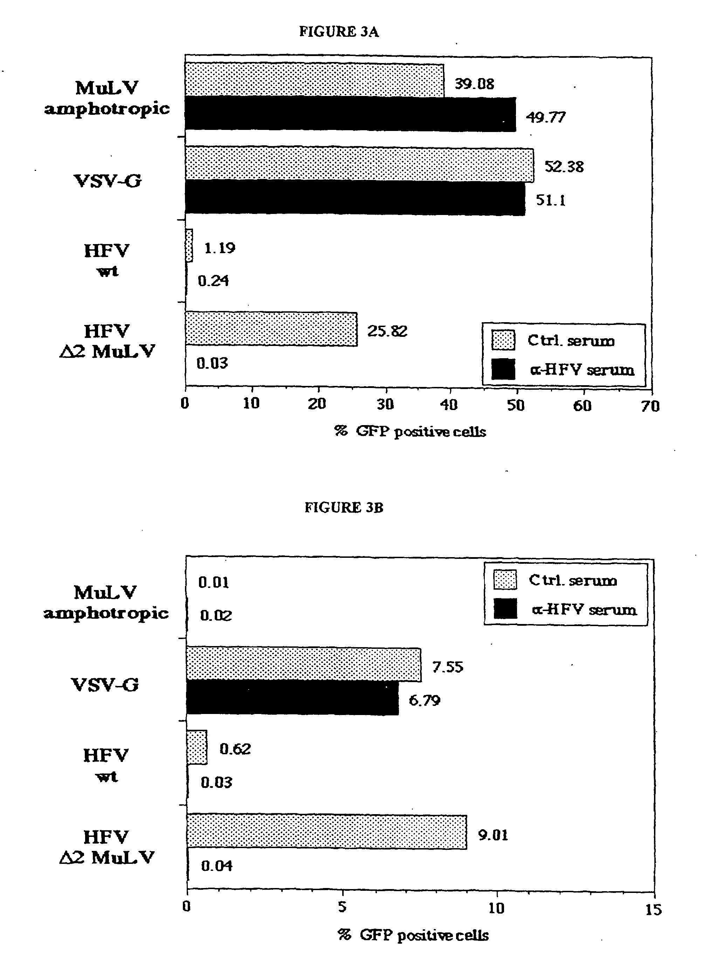

To confirm that the infectivity of MuLV particles pseudotyped with different HFV envelope proteins was specific for the HFV envelope, pseudotyped particles were preincubated with an anti-HFV specific chimpanzee serum prior to the addition to target cells (3). The infectivity of viral particles pseudotyped with the amphotropic MuLV envelope or the VSV-G protein was not reduced by preincubation with the HFV specific antiserum when compared to the preincubation of these viruses with normal heat inactivated human serum (Fig. 3) or mock incubated viral particles (data not shown). In contrast, the infectivity of particles pseudotyped with the wildtype HFV envelope protein or the HFV Δ2MuLV chimera was completely abolished by the preincubation with the HFV specific antiserum but not the human control serum (Fig. 3). This neutralization ofHFV envelope specific infectivity was observed for NIH3T3 (Fig. 3A) and QT-6 (Fig. 3B) cells. A similar specific neutralization of viral particles pseudotyped with HFV envelope proteins was obtained in experiments using a rabbit serum raised against the baculovirus expressed SU domain of the HFV envelope protein (data not shown).

### 4. Expression and particle incorporation of HFV env proteins

The expression and incorporation of the different HFV envelope proteins into MuLV particles was determined by radioimmunoprecipitation analysis (RIPA) of transiently transfected 293T cells. Forty-eight hours after addition of the DNA (pHIT60, SFG GFPS65T and various env constructs), cells were metabolically labeled with [³⁵S]methionine for approximately 20 h. Viral particles present in the supernatant were pelleted by centrifugation at 25 000 rpm through a 20 % sucrose cushion prior to solubilization in lysis buffer. Subsequently, the samples were submitted to immunoprecipitation. Immunoprecipitates of the viral particles with an HFV specific chimpanzee serum or antiMuLV gag hybridoma supernatants were analyzed by SDS-polyacrylamide gel electrophoresis (PAGE) along with their corresponding cell lysates. HFV specific bands in immunoprecipitates from pelleted virus or cellular lysates were only observed in samples transfected with the HFV env expression constructs, but not in samples expressing the MuLV amphotropic envelope protein or mock transfected cultures. Two predominant HFV envelope precursor bands of 130 and 110 KD were observed in immuno-precipitates of cellular lysates of HFV env transfected cells (12, 21). In addition, two bands corresponding to the processed ∼90 KD SU and the ∼45-50 KD TM proteins could be observed after longer exposure. The different apparent sizes of the TM proteins in the cellular samples transfected with the various HFV mutants reflected the modifications in the TM portion of the individual proteins. Only moderate differences in the steady state level of the different envelope proteins in the transfected cells were observed, except for the HFV SSSMuLVR- and the HFV SSSMuLV proteins which showed a clearly reduced cellular expression. Both envelope precursor proteins as well as the processed SU and TM proteins were also detected in immunoprecipitates of pelleted viral particles. However, in general the relative ratio of processed proteins to precursor proteins was increased in the viral particle immunoprecipitates compared to the cell lysates.

Interestingly, a good correlation between the amount of processed SU and TM proteins in the individual immunoprecipitates of the viral particles and the relative infectivity of the corresponding pseudotyped particles (Fig. 2) could be observed. The HFV Δ2MuLV chimeric envelope, which gave rise to pseudotyped particles with the highest relative infectivity also showed the strongest SU and TM bands in the RIPA. The amount of MuLV gag/pol proteins in the individual viral particle preparations, as determined in crude viral pellets or immunoprecipitates with anti-gag hybridoma supernatants, was similar for all samples, except for the HFV Δ2MuLV envelope transfection. This sample showed a significant decrease in particle associated gag/pol proteins, indicating that fewer MuLV particles were present in this preparation compared to the other viral pellets. As a result, the relative amount of processed HFV SU and TM proteins per individual viral particle may be even higher than estimated from the immunoprecipitates of viral particles preparations with HFV specific antibodies. A possible explanation for this phenomenon is an enhanced absorbance of these particles by transfected cells not expressing the HFV env protein, as a result of the increased infectivity of HFV Δ2MuLV pseudotyped particles compared to particles pseudotyped by the other HFV envelope proteins. This may result in a clearing of the HFV Δ2MuLV pseudotyped particles from the supernatant. Furthermore, in contrast to MuLV particles pseudotyped with amphotropic MuLV envelope or VSV-G, HFV pseudotyped viruses showed no reduction in infectivity in the absence of polycations such as polybrene (30). Therefore, the relative titers of retroviral vectors pseudotyped with HFV envelope by transient transfection may be underestimated compared to pseudotypes with amphotropic MuLV envelope or VSV-G. Further experiments, however, using cell lines stably expressing the HFV envelope, which should be resistant to infection by viruses pseudotyped with the HFV envelope, are necessary to clarify these phenomena in more detail.

### 5. Inactivation of the splice donor and acceptor sites located into the FV env gene

Furthermore, Bel-1 and Bet transcripts derived from internal HFV promotor (Pos. 8419 relative to the transcription start in the 5' LTR), located within the HFV envelope ORF (Pos. 6310-9276) efficiently utilize a splice donor (SD, Pos. 9119 and a splice acceptor (SA, Pos. 9237) site within the coding region ofTM subunit of the env protein. Alternative splicing of mRNA coding for the HFV env protein utilizing these SD and SA sites results in potential envelope/bel fusion proteins. A ∼170 KD can be detected in HFV infected cells by immunoprecipitation and the mRNA is detectable by RT (reverse-transcriptase) PCR of total mRNA from HFV infected human fibroblasts. Inactivation of the SD (Pos. 9119) by a GT->GG mutation results in a disapearance of the 170 KD envelope fusion protein, while the expression of the 130 KD envelope precursor protein is not changed. The biological function of the env/bel fusion proteins as well as the influence on viral titers of pseudotyped MuLV particles are currently not known.

In summary, a system has been generated to produce MuLV based retroviral vectors pseudotyped with HFV envelope proteins. The cytoplasmic domain of the HFV envelope protein was at least partially involved in pseudotyping of MuLV particles as progressive deletion of the cytoplasmic domain lead to a reduction in gene transfer and incorporation of HFV env SU and TM subunits into the viral particle. Addition of an unprocessed MuLV envelope cytoplasmic domain to one deletion mutant, the HFV Δ2MuLV envelope, resulted in a 10-20 fold increase in infectivity compared to HFV wildtype envelope protein and an enhanced incorporation of the chimeric envelope protein into pseudotyped particles. Retroviral titers were 8-35 fold higher than those achieved by pseudotyping with the wildtype HFV envelope protein. On some target cell types, the gene transfer efficiency was similar or higher than those of retroviral vectors pseudotyped with the VSV-G protein. In the case of the wildtype MuLV envelope protein, the role of the cytoplasmic domain for the specific incorporation of the envelope into the viral particle is unclear. Some cytoplasmic tail deletion mutants resulted in a loss of particle associated envelope proteins (15), whereas other mutant envelope proteins showed little to no reduction in particle association (25, 26). Our results argue for a role of the MuLV env cytoplasmic domain in the particle association of the envelope protein, at least in the enhanced incorporation of chimeric envelope proteins into MuLV particles.

Recently, the pseudotyping of MuLV based retroviral vectors with foreign envelope proteins, such as the VSV glycoprotein G (6, 38) or the GALV envelope (2, 34), has resulted in an increase in virus stability, a broadened host-cell range and an enhanced transduction efficiency of certain cell types. The broad host range of FVs, the resistance to inactivation by human serum (30), and the efficient infection of cells of various origin in the absence of polycations (unpublished observations and (30)) should make MuLV based retroviral vectors pseudotyped with the HFV Δ2MuLV chimeric envelope protein a useful new tool for efficient gene transfer into different cell types. Unlike the expression of VSV-G, which is highly toxic for the producer cells and has prevented the generation of stable VSV-G packaging cell lines until recently (8, 22, 37), transient expression of the HFV Δ2MuLV envelope resulted in no apparent toxicity in 293T cells (data not shown, (19)).

### EXAMPLE 2: HFV/SIV env chimeras

This example describes the construction of chimeric envelopes betweeen HFV and SIV envelope proteins. Three different types of chimeric envelopes were constructed. the sequence of the different elements used herein after are available in Genbank : SIV mm251 genome under accession number M19499, CMV promoter under X03922 and pEGFP-C1 (GFP gene) under U55763.

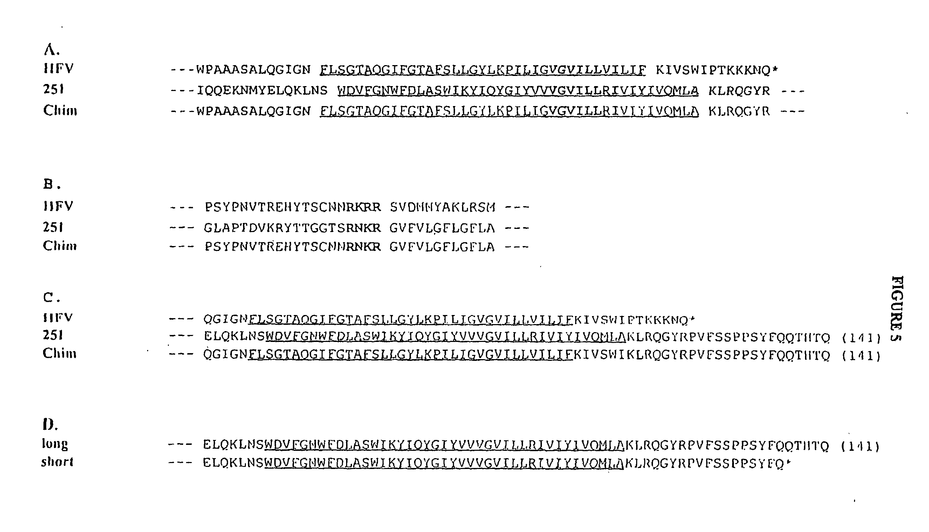

In the first type, the cytoplasmic tail of SIV is fused to the extracellular domain (also designated in the literature ectodomain) of HFV within the transmembrane anchor domain (constructs 1, 2 and 7 of Figure 4 and Figure 5A). The transmembrane anchor domain of the HFV env is deduced from its amino acid sequence, and is therefore hypothetical. The transmembrane anchor domain of the SIV env protein contains a small region of identical amino acids with respect to HFV env. It is in this region that the fusion has been made. Thus the entire extracellular domain of the chimeric env gene is from HFV, including the entire surface subunit (SU) and most of the transmembrane subunit (TM). The cytoplasmic tail is entirely from SIV.

In the second type of chimeric envelopes, the fusion genes is at the level of the cleavage site between the SU and the TM subunits (constructs 3, 4 and 8 of Figure 4 and Figure 5B). Thus the SU subunit is from HFV and the TM subunit from SIV. In the chimeric envelope protein, the cleavage site of the SIV env (Arg-Gln-Lys-Arg) was used.

In the third type of chimeric envelope, the SIV cytoplasmic tail is fused to the HFV envelope in the cytoplasmic domain (constructs 5, 6 and 9 of Figure 4 and Figure 5C). This construct is similar in design to the chimeric envelope Δ2MuLV and retains the first 6 cytoplasmic amino acids from the HFV envelope. The entire cytoplasmic tail from SIV is fused to this envelope protein.

There exists two versions of the SIV envelope gene (see Figure 5D and reference 42) : a long form having a tail of 164 amino acids which is present in SIV particles replicating in the natural host, the rhesus monkey (Macaca mulatta) and a short form nammed «humanized» containing only 18 amino acids. This short form is selected for when virus isolated from the monkey are cultured on human cell lines. Chimeric envelopes have been constructed using both tails. Constructs 1, 3 and 5 comprise the long version and the remaining constructs the short version.

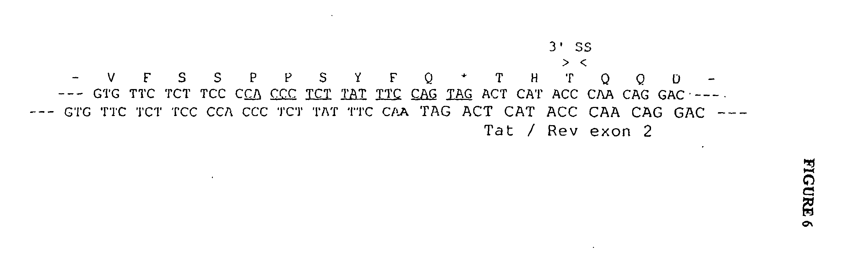

The possible presence of cis-acting repressor sequences (CRS) in the SIV envelope genes may retain the env messenger RNA in the nucleus of producer cells. The inhibitory effect on expression of env genes can be overcome if these messenger RNAs contain the rev responsive element (RRE) and if, at the same time, rev is expressed. This is the case for the second type of chimeric envelope constructs since the RRE is present in the TM subunit of SIV and rev will be expressed from a plasmid containing the SIV proviral genome. However, the RRE is not present in the other envelope constructs. Although the exact nature of the CRS sequences in the SIV env gene is not known, it is possible that the 3' splice site of the second tat/rev exon in the env gene constitutes a CRS. We therefore decided to construct a third variant of the chimeric env constructs in which this 3' splice site was destroyed (43, 44, Figure 6). Destruction of the 3' splice site was only done in chimeric envelopes with the short, «humanized» cytoplasmic tail from SIV env.

In total 9 chimeric envelopes have been constructed (Figure 4):
Env 1, 2 and 7 where fusion is in the trans membrane anchor domain, Env 1 has a long SIV cytoplasmic tail, Env 2 and 7 a short one. The 3' splice site in the env gene is present in env 2 but has been deleted in env 7. The amino acid sequence of Env 2 and 7 are identical.
Env 3, 4 and 8 where fusion is at the cleavage site, Env 3 has a long cytoplasmic tail, Env 4 and 8 a short one. The 3' splice site in the env gene is present in env 4 but has been deleted in env 8. The amino acid sequence of Env 4 and 8 are identical.
Env 5, 6 and 9 where fusion is in the cytoplasmic tail, Env 5 has a long cytoplasmic tail, Env 6 and 9 a short one. The 3' splice site in the env gene is present in env 6 but has been deleted in env 9. The amino acid sequence of Env 6 and 9 are identical.

### 1. Construction of the chimeric env genes

All chimeric env genes have been cloned by replacing the appropriate HFV env sequences in plasmid pczHFVenv wt with sequences from the SIV env gene. pczHFVenv wt contains the HFV wild type env encoding sequences cloned in pcDNA3.1/Zeo (Invitrogene) under the control of the CMV promoter. SIV env sequences were amplified from a plasmid containing the long version of the env gene (pTG 664) for envelope constructs 1, 3 and 5 and from a plasmid containing the short version of the env gene (pTG 626Sma+) for the remaining constructs. These two plasmids may be generated by a man skilled in the art by cloning SIV env versions into p poly III*I (45). Amplification was carried out with an upstream primer having a 5' extension of 50 nucleotides from the HFV env gene, and a downstream primer having a 3' extension of 15 nucleotides containing recognition sites for the restriction enzymes XhoI and EcoRI. Both primers have a region of 20 nucleotides complementary to the SIV env gene permitting amplification of specific parts of the SIV env genes.

After PCR of C-terminal parts of the SIVenv gene, amplimers were isolated from gel and digested with Xho I to remove the 3' terminal nucleotides from the amplimer. Plasmid pczHFVenv wt was digested with XhoI and EcoRI. The amplimers were ligated to the linear pczHFVenv wt plasmid using the XhoI redundant ends. This results in a linear DNA fragment containing the entire HFV env gene and the cytoplasmic tail of SIV. The 5' end of the cytoplasmic tail contains sequences homologous to the HFV env gene that is recombined by transformation of this plasmid in E. coli BJ 5183 cells (46) according to the technology described in (41). This results in a closed circular plasmid in which the 3' terminal part of HFV env has been replaced by the 3' end of SIV env. Using different combinations of primers, all chimeric envelopes have been constructed this way (see Table 2). In the resulting plasmid, the chimera encoding sequences are placed under the control of the immediate early CMV promoter.

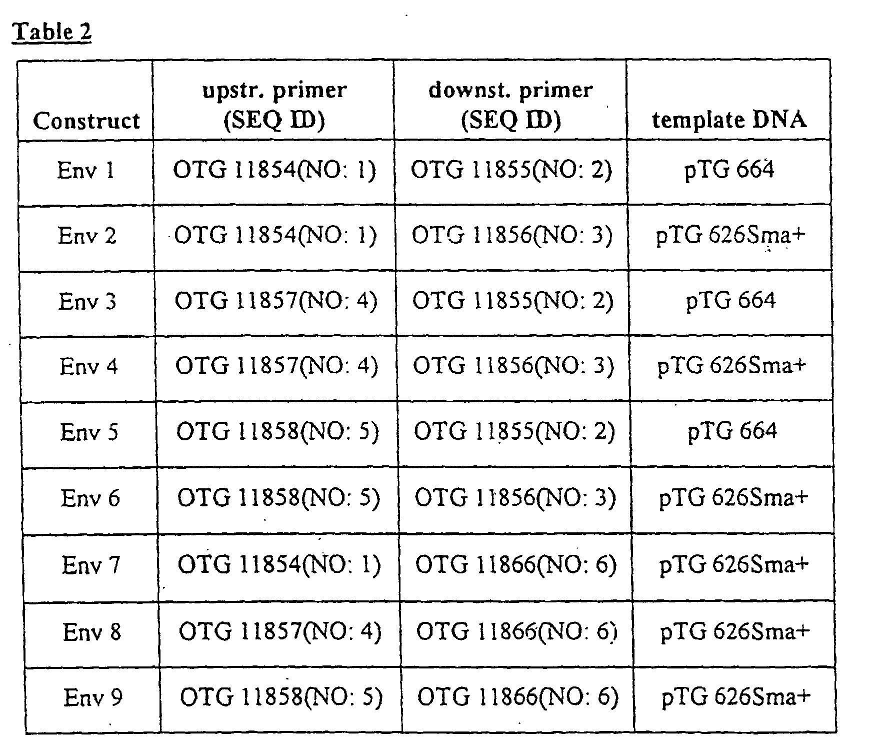

### 2. Production of pseudotyped SIV particles and transduction of target cells.

Production of pseudotyped retroviral particles is achieved by the co-transfection of 293 cells with a the different plasmids expressing the envelope chimeras and a plasmid containing the proviral SIV genome in which env gene is non functional due to the insertion of an expression cassette consisting of the immediate early CMV promoter and the gene coding for the Enhanced Green Fluorescent Protein (GFP)(nt 613 to 1330 of Genbank sequence U55763).

The protocol is the following : 293 cells were plated in 10 cm petri dishes at a density of 2 x 10⁶ per dish in DMEM medium complemented with 10 % fetal calf serum, non-essential amino acids, gentamycin and glutamine (complete DMEM). The next day, the cells were transfected using the standard Calcium Phosphate transfection technique with 25 µg SIV proviral plasmid and 5 µg envelope plasmid (the 9 chimeric envelope expression plasmids or control plasmids: empty expression plasmid ; VSV-G protein expression plasmid or pczHFVenv WT). The next day, medium was removed and cells were washed once in DMEM medium complemented with 5 % fetal calf serum, non-essential amino acids, gentamycin and glutamine and then incubated in 6 ml of the same medium. Virus containing medium was harvested two days later and cleared by centrifugation (5 min. at 3500 rpm). Target cells (293 or HT 1080) which were seeded the previous day at a density of 5 x 10⁵ cells per well of a 6-well plate were transduced as follows: target cells were washed with 1 ml of DMEM (no serum), and then covered with 300 µl DMEM. 300 µl virus containing supernatant supplemented with 10 µg protamine sulfate/ml was added and cells were incubated for 2 hours at 37 °C in a 5% CO₂ atmosphere. After addition of 3 ml complete DMEM incubation was continued for 3 days.

Producer and target cells were analysed by FACScan as follows: cells were trypsinized and washed with PBS. Cells were fixed with 1 ml PBS/4 % formaldehyde for 10 minutes at 4 °C. After one more wash step, cells were resuspended in 1 ml PBS and analysed with a Becton-Dickinson FACScan using Cell-Quest software. Fluorescence was measured using the FITC filter. All producer cells were transfected with high efficiency. Transduction of control target cells was as expected: VSV-G pseudotyped particles are able to transduce 293 cells and HT 1080 cells. Supernatants from transfected cells without envelope protein or particles pseudotyped with HFV env were not able to transduce any target cells. Viral particles produced by the constructs 6 and 9 are able to transduce 293 and HT 1080 cells, showing that their chimeric envelope is functional.

### References

1/ Adams, R. M., H. E. Soriano, M. Wang, G.Darlington, D. Steffen, and F. D. Ledley. 1992. Transduction of primary human hepatocytes with amphotropic and xenotropic retroviral vectors. Proc Natl Acad Sci U S A 89 : 8981-5.
2/ Bauer, T., Jr., A. D. Miller, and D. D. Hickstein. 1995. Improved transfer of the leukocyte integrin CD 18 subunit into hematopoietic cell lines by using retroviral vectors having a gibbon ape leukemia virus envelope. Blood 86 : 2379-87.
3/ Bieniasz, P. D., A. Rethwilm, R. Pitman, M. D. Daniel, I. Chrystie, and M. O. McClure. 1995. A comparative study of higher primate foamy viruses, including a new virus from a gorilla. Virology 207 : 217-28.
4/ Bieniasz, P. D., R A. Weiss, and-M. O. McClure. 1995. Cell cycle dependence of foamy retrovirus infection. J. Virol. 69 : 7295-7299.
5/ Bueler, H., and R. C. Mulligan. 1996. Induction of antigen-specific tumor immunity by genetic and cellular vaccines against MAGE : enhanced tumor protection by coexpression of granulocyte-macrophage colony- stimulating factor and B7-1. Mol. Med. 2 : 545-555.
6/ Bums, J. C., T. Friedmann, W. Driever, M. Burrascano, and J. K. Yee. 1993. Vesicular stomatitis virus G glycoprotein pseudotyped retroviral vectors : concentration to very high titer and efficient gene transfer into mammalian and nonmammalian cells. Proc Natl Acad Sci U S A 90 : 8033-7.
7/ Chalfie, M., Y. Tu, G. Euskirchen, W. W. Ward, and D. C. Prasher. 1994. Green fluorescent protein as a marker for gene expression Science 263 : 802-5.
8/ Chen, S. T., A. Iida, L. Guo, T. Friedmann, and J. K. Yee. 1996. Generation of packaging cell lines for pseudotyped retroviral vectors of the G protein of vesicular stomatitis virus by using a modified tetracycline inducible system. Proc. Natl Acad Sciences USA 93:10057-10062.
9/ Du Bridge, R. B., P. Tang, H. C. Hsia, P. M. Leong, J. H. Miller, and M. P. Calos. 1987. Analysis of mutation in human cells by using an Epstein-Barr virus shuttle system. Mol Cell Biol 7 : 37987.
10/ Enssle, J., I. Jordan, B. Mauer, and A. Rethwilm. 1996. Foamy virus reverse transcriptase is expressed independently from the gag protein. Proc Natl. Acad. Sci. (USA) 93 : 4137-4141.
11/ Flügel, R. M., A. Rethwilm, B. Maurer, and G. Darai. 1987. Nucleotide sequence analysis of the *env* gene and its flanking regions of the human spumaretrovirus reveals two novel genes EMBO 6 : 2077-2084.
12/ Giron, M. L., F. Rozain, M. C. Debons-Guillemin, M. Canivet, J. Peries, and R. Emanoil-Ravier. 1993. Human foamy virus polypeptides : identification of env and bel gene products J. Virol 67 : 3596-600.
13/ Goepiert, p. A., K. L. Shaw, G. D. J. Ritter, and M. J. Mulligan. 1996. A sorting motif localizes the foamy virus glycoprotein to the endoplasmic reticulum. J. Virol. 71 : 778-784.
14/ Goepfert, P. A., G. Wang, and M. J. Mulligan. 1995. Identification of an ER retrieval signal in a retroviral glycoprotein. Cell 82 : 543-544.
15/ Granowitz, C., J. Colicelli, and S. P. Goff. 1991. Analysis of mutations in the envelope gene of Moloney Murine Leukemia Virus : Separation of infectivity from superinfections Resistance. Virology 183 : 545-554.
16/ Green, N., T. M. Shinnick, O. Witte, A. Ponticelli, J. G. Sutcliffe, and R. A. Lerner. 1981. Sequence-specific antibodies show that maturation of Moloney leukemia virus envelope polyprotein involves removal of a COOH-terminal peptide. Proc. Natl. Acad. Sci. USA 78 : 6023-6027.
17/ Henderson, L. E., R. Sowder, T. D. Copeland, G. Smythers, and S. Oroszlan. 1984. Quantitative separation of murine leukemia virus proteins by reversed-phase high pressure liquid chromatography reveals newly described gag and *env* cleavage products. J. Virol. 52:492-500.
18/ Jordan, I., J. Enssle, E. Guttler, B. Mauer, and A. Rethwilm. 1996. Expression of human foamy virus reverse transcriptase involves a spliced pol mRNA. Virology 224 : 314-319.
19/ Mikovits, J. A., P. M. Hoffman, A. Rethwilm, and F. W. Ruscetti. 1996. In vitro infection of primary and retroviral infected human leukocytes by human foamy virus. J. Virol. 70 : 2774-2780.
20/ Miller, A. D. 1992 Human gene therapy comes of age. Nature 357 : 455-60.
21/ Netzer, K. 0., A. Rethwilm, B: Maurer, and V. ter Meulen. 1990. Identification of the major immunogenic structural proteins of human foamy virus. J Gen Virol. 71 : 1237-41.
22/ Ory, D. S., B. A. Neugeboren, and R. C. Mulligan. 1996 A stable human-derived packaging cell line for production of high titer retrovirus/vesicular stomatitis virus G pseudotypes. Proc. Natl Acad Sciences USA 93 : 11400-11406.
23/ Owens, R. J., and J. K. Rose. 1993. Cytoplasmic domain requirement for incorporation of a foreign envelope protein into vesicular stomatitis virus. J. Virol. 67 : 360-5.
24/ Ragheb, J. A., and W. F. Anderson. 1994. pH-independent Murine Leukemia Virus ecotropic envelope-mediated cell fusion : Implications for the role of the R peptide and p12E TM in viral entry. J. Virol. 68 : 3220-3231.
25/ Ragheb, J. A., and W. F. Anderson. 1994. Uncoupled expression of Moloney Murine Leukemia Virus envelope polypeptides SU and TM : a functional analysis of the role of TM domains in viral entry. J. Virol. 68 : 3207-3219.
26/ Rein, A, A. Mirro, J. Gordon Haynes, S. M. Ernst, and K. Nagashima. 1994. Function of the cytoplasmic domain of a retroviral transmembrane protein : p15E-p2E cleavage activates the membrane fusion capability of the Murine Leukemia Virus env protein. J. Virol. 68 : 1773-1781.
27/ Rethwilm, A. 1996. Unexpected replication pathways of foamy viruses. J. Acquired Immune Defic. Syndr. Hum. Retrovirol.
28/ Rethwilm, A., G. Baunach, K. O. Netzer, B. Maurer, B. Borisch, and V. T. Meulen. 1990. Infectious DNA of the human spumaretrovirus. Nucleic Acids Res. 18 : 733-738.
29/ Rose, J. K., and J. E. Bergmann. 1983. Altered cytoplasmic domains affect intracellular transport of the vesicular stomatitis virus glycoprotein. Cell 34 : 513-524.
30/ Russel, D. W., and A. D. Miller. 1996. Foamy virus vectors. J. Virol. 70 : 217-222.
31/ Schliephake, A. W., and A. Rethwilm. 1994. Nuclear localization of foamy virus gag precursor protein. J. Virol. 68 : 4946-4954.
32/ Schmidt, M., and A. Rethwilm. 1995. Replicating foamy virus-based vectors directing high level expression of foreign genes. Virology 210 : 167-78.
33/ Soneoka, Y., P. M. Cannon, E. E. Ramsdale, J. C. Griffiths, G. Romano, S. M. Ringsman, and A. J. Kingsman. 1995. A transient three-plasmid expression system for the production of high titer retroviral vectors. Nucleic Acids Res 23 : 628-33.
34/ von Kalle, C., H. P. Kiem, S. Goehle, B. Darovsky, S. Heinfeld, B. Torok-Storb, R. Storb, and F. G. Schuening. 1994. Increased gene transfer into human hematopoietic progenitor cells by extended in vitro exposure to a pseudotyped retroviral vector. Blood 84 : 2890-7.
35/ Weiss, R. A. 1996. Foamy viruses bubble on. Nature 380 : 201.
36/ Yang, C., and R. W. Compans. 1996. Analysis of the cell fusion activities of chimeric simian immunodeficiency virus-murine leukemia virus envelope proteins: inhibitory effects of the R peptide. J. Virol. 70: 248-254.
37/ Yang, Y., E. F. Vanin, M. A. Whitt, M. Fomerod, R: Zwart, R. D. Schneiderman, G. Grosveld, and A. W. Nienhuis. 1995. Inducible, high-level production of infectious murine leukemia retroviral vector particles pseudotyped with vesicular stomatitis virus G envelope protein. Hum Gene Ther 6 : 1203-13.
38/ Yee, J. K., A. Miyanohara, P. LaPorte, K. Bouic, J. C. Burns, and T. Friedmann. 1994. A general method for the generation of high-titer, pantropic retroviral vectors. Highly efficient infection of primary hepatocytes Proc Natl Acad Sciences USA 91 : 9564-9568.
39/ Yu, S. F., D. N. Baldwin, S. R. Gwynn, S. Yendapalli, and M. L. Linial. 1996. Human foamy virus replication : a pathway distinct from that of retroviruses and hepadnaviruses. Science 271 : 15791582.
40/ Yu, S. F., K. Edelmann, R. K. Strong, A. Moebes, A. Rethwilm, and M. L. Linial. 1996. The carboxyl terminus of the human foamy virus gag protein contains separable nucleic acid binding and nuclear transport domains. J. Virol. 70 : 8355-8262.
41/ Chartier C., Degryse, E., Gantzer, M., Dieterle, A., Pavirani, A. and Mehtali, M. 1996. Efficient generation of recombinant adenovirus vectors by homologous recombination in Escherichia coli. J. Virol. 70, 4805-4810.
42/ Zingler, K. and Littman, D.R. 1993. Truncation of the cytoplasmic domain of the simian immunodeficiency virus envelope glycoprotein increases env incorporation into particles and fusogenicity and infectivity. J. Virol. 67, 2824-2831
43/ Hammarskjöld, M.L., Li, H., Rekosh, D. and Prasad, S. 1994. human immunodeficiency virus env expression becomes Rev-independent if the env region is not defined as an intron. J. Virol. 68, 951-958.
44/ Chang, D.D. and Sharp, P.A. 1989. Regulation by HIV rev depends upon recognition of splice sites. Cell 59; 789-795.
45/ Lathe, R. et al., 1987. Gene 57, 193-201.
46/ Hananan, 1983, J. Mol. Biol. 166, 557-58.

## Claims

1. Construct for the expression of a protein comprising at least a modified foamy virus (FV) envelope protein for the production of pseudotyped viral particles, wherein the modification is at least a truncation at residue 975 or 981 of said protein, **characterized in that** said protein is a fusion protein furthermore comprising all or part of a non-FV envelope protein.

2. Construct for the expression of a protein according to claim 1, **characterized in that** the foamy virus is the human foamy virus (HFV).

3. Construct for the expression of a protein according to claim 1 or 2, **characterized in that** the non-FV envelope protein is derived from a virus selected in the group comprising MuLV, MoMuLV, FB 29, HIV and SIV.

4. Construct for the expression of a protein according to claim 3, **characterized in that** said fusion is within the transmembrane anchor domain of said FV and non-FV envelope proteins.

5. Construct for the expression of a protein according to claim 4, **characterized in that** the protein comprises the extracellular domain and the 5' part of the transmembrane anchor domain of the HFV envelope protein and the 3' part of the transmembrane anchor domain and the cytoplasmic domain of the non-FV envelope protein, particularly of the SIV envelope protein.

6. Construct for the expression of a protein according to claim 3, **characterized in that** said fusion is within the cleavage site of said FV and non-FV envelope proteins.

7. Construct for the expression of a protein according to claim 6, **characterized in that** the protein comprises the SU domain and all or part of the cleavage site of the HFV envelope protein and all or part of the cleavage site and the TM domain, comprising the transmembrane anchor domain and the cytoplasmic domain, of the non-FV envelope protein, particularly of the SIV envelope protein.

8. Construct for the expression of a protein according to claim 3, **characterized in that** said fusion is at the junction between the transmembrane anchor domain and the cytoplasmic domain or within the cytoplasmic domains of said FV and non-FV envelope proteins.

9. Construct for the expression of a protein according to claim 8, **characterized in that** the protein comprises the extracellular domain, the transmembrane anchor domain and all or part of the cytoplasmic domain of the HFV envelope protein and all or part of the cytoplasmic domain of the non-FV envelope protein, particularly of the SIV envelope protein.

10. Construct for the expression of a protein according to anyone of claims 1 to 9, **characterized in that** the protein consists in the HFV envelope protein which all or part of the cytoplasmic domain is replaced by all or part of a non-FV viral envelope protein, and preferably ofMuLV retroviral envelope protein.

11. Construct for the expression of a protein according to anyone of claims 1 to 10, **characterized in that** the protein consists in the fusion of all or part of a MuLV cytoplasmic domain to a modified HFV envelope protein.

12. Construct for the expression of a protein according to claims 10 and 11, **characterized in that** the MuLV cytoplasmic domain is processed or unprocessed.

13. Construct for the expression of a protein according to anyone of claims 1 to 12, **characterized in that** the protein comprises a modified HFV envelope protein truncated of residue 981 which is fused to an arginine residue and the unprocessed MuLV cytoplasmic domain extending from residue 634 to residue 665 of the wild type MuLV envelope protein.

14. Construct for the expression of a protein according to anyone of claims 1 to 13, **characterized in that** the construct comprises a mutation of the donor and/or the acceptor splicing site(s) naturally present in the FV envelope protein encoding sequence.

15. Protein as expressed by a construct according to anyone of claims 1 to 14.

16. Pseudotyped viral particle comprising a protein according to claim 15.

17. Complementation cell line comprising a construct according to anyone of claims 1 to 14.

18. Complementation cell line according to claim 17, comprising a construct according to claim 9 or 13.

19. Complementation cell line according to claim 17 or 18, further comprising a construct expressing a viral gag / pol gene.

20. Complementation cell line according to claim 19, **characterized in that** the retroviral gag / pol gene derives from MuLV or FB 29 or SIV.

21. Complementation cell line according to anyone of claims 17 to 20, **characterized in that** the cell line derives from the 293 line.

22. Method for the preparation of pseudotyped viral particle according to claim 16, which comprises:
(i)introducing a recombinant retroviral vector into a complementation cell according to anyone of claims 17 to 21,
(ii) culturing said complementation cell line under suitable conditions to permit the production of the said pseudotyped viral particle, and
(iii) recovering said pseudotyped viral particle from the cell culture.

23. Mammalian cell infected with a pseudotyped viral particle according to claim 16 or with a pseudotyped viral particle obtainable by a method according to claim 22.

24. Pharmaceutical composition comprising a therapeutically or prophylactically effective amount of a pseudotyped viral particle according to claim 16 or obtainable by a method according to claim 22 or a mammalian cell according to claim 23.

25. Use of a pseudotyped viral particle according to claim 16 or obtainable by a method according to claim 22 or a mammalian cell according to claim 23 for preparing a drug intended for the treatment of a genetic disorder, a cancer or a virally-induced disease.

26. Use of a construct as defined in anyone of claims 1 to 14 for the expression of a protein for the production ofpseudotyped viral particles, said protein comprising at least a modified foamy virus (FV) envelope protein and said modification being at least a truncation at residue 975 or 981 of said protein.

27. Use of a construct according to claim 26, **characterized in that** the foamy virus is the human foamy virus (HFV).

## Patentansprüche

1. Konstrukt für die Expression eines Proteins, welches wenigstens ein modifiziertes Hüllprotein eines Foamy-Virus (FV) umfasst, für die Herstellung von pseudotypisierten Viruspartikeln, wobei die Modifikation zumindest eine Verkürzung an Rest 975 oder 981 des Proteins ist, **dadurch gekennzeichnet, dass** das Protein ein Fusionsprotein ist, welches darüber hinaus die Gesamtheit oder einen Teil eines Nicht-FV-Hüllproteins umfasst.

2. Konstrukt für die Expression eines Proteins nach Anspruch 1, **dadurch gekennzeichnet, dass** das Foamy-Virus das humane Foamy-Virus (HFV) ist.

3. Konstrukt für die Expression eines Proteins nach Anspruch 1 oder 2, **dadurch gekennzeichnet, dass** das Nicht-FV-Hüllprotein von einem Virus abgeleitet ist, welches ausgewählt wird in der Gruppe, umfassend MuLV, MoMuLV, FB 29, HIV und SIV.

4. Konstrukt für die Expression eines Proteins nach Anspruch 3, **dadurch gekennzeichnet, dass** die Fusion innerhalb der Transmembranankerdomäne der FV- und Nicht-FV-Hüllproteine erfolgt.

5. Konstrukt für die Expression eines Proteins nach Anspruch 4, **dadurch gekennzeichnet, dass** das Protein die extrazelluläre Domäne und den 5'-Abschnitt der Transmembranankerdomäne des HFV-Hüllproteins und den 3'-Abschnitt der Transmembranankerdomäne und die zytoplasmatische Domäne des Nicht-FV-Hüllproteins, insbesondere des SIV-Hüllproteins, umfasst.

6. Konstrukt für die Expression eines Proteins nach Anspruch 3, **dadurch gekennzeichnet, dass** die Fusion innerhalb der Spaltstelle der FV- und Nicht-FV-Hüllproteine erfolgt.

7. Konstrukt für die Expression eines Proteins nach Anspruch 6, **dadurch gekennzeichnet, dass** das Protein die SU-Domäne und die Gesamtheit oder einen Teil der Spaltstelle des HFV-Hüllproteins und die Gesamtheit oder einen Teil der Spaltstelle und die TM-Domäne, umfassend die Transmembranankerdomäne und die zytoplasmatische Domäne, des Nicht-FV-Hüllproteins, insbesondere des SIV-Hüllproteins, umfasst.

8. Konstrukt für die Expression eines Proteins nach Anspruch 3, **dadurch gekennzeichnet, dass** die Fusion an der Verbindungsstelle zwischen der Transmembranankerdomäne und der zytoplasmatischen Domäne oder innerhalb der zytoplasmatischen Domänen der FV- und Nicht-FV-Hüllproteine erfolgt.

9. Konstrukt für die Expression eines Proteins nach Anspruch 8, **dadurch gekennzeichnet, dass** das Protein die extrazelluläre Domäne, die Transmembranankerdomäne und die Gesamtheit oder einen Teil der zytoplasmatischen Domäne des HFV-Hüllproteins und die Gesamtheit oder einen Teil der zytoplasmatischen Domäne des Nicht-FV-Hüllproteins, insbesondere des SIV-Hüllproteins, umfasst.

10. Konstrukt für die Expression eines Proteins nach einem der Ansprüche 1 bis 9, **dadurch gekennzeichnet, dass** das Protein aus dem HFV-Hüllprotein besteht, bei dem die Gesamtheit oder ein Teil der zytoplasmatischen Domäne durch die Gesamtheit oder einen Teil eines Hüllproteins eines Nicht-FV-Virus, und vorzugsweise eines Hüllproteins eines MuLV-Retrovirus, ersetzt ist.

11. Konstrukt für die Expression eines Proteins nach einem der Ansprüche 1 bis 10, **dadurch gekennzeichnet, dass** das Protein aus der Fusion der Gesamtheit oder eines Teils einer zytoplasmatischen MuLV-Domäne an ein modifiziertes HFV-Hüllprotein besteht.

12. Konstrukt für die Expression eines Proteins nach den Ansprüchen 10 und 11, **dadurch gekennzeichnet, dass** die zytoplasmatische MuLV-Domäne prozessiert oder unprozessiert ist.

13. Konstrukt für die Expression eines Proteins nach einem der Ansprüche 1 bis 12, **dadurch gekennzeichnet, dass** das Protein ein modifiziertes, um Rest 981 verkürztes HFV-Hüllprotein, welches mit einem Argininrest fusioniert ist, und die unprozessierte zytoplasmatische MuLV-Domäne, welche sich von Rest 634 bis Rest 665 des Wildtyp-MuLV-Hüllproteins erstreckt, umfasst.

14. Konstrukt für die Expression eines Proteins nach einem der Ansprüche 1 bis 13, **dadurch gekennzeichnet, dass** das Konstrukt eine Mutation der Donor- und/oder der Akzeptor-Spleißstelle(n), welche von Natur aus in der das FV-Hüllprotein kodierenden Sequenz vorhanden ist bzw. sind, umfasst.

15. Protein, wie es durch ein Konstrukt nach einem der Ansprüche 1 bis 14 exprimiert wird.

16. Pseudotypisiertes Viruspartikel, umfassend ein Protein nach Anspruch 15.

17. Komplementationszelllinie, welche ein Konstrukt nach einem der Ansprüche 1 bis 14 umfasst.

18. Komplementationszelllinie nach Anspruch 17, welche ein Konstrukt nach Anspruch 9 oder 13 umfasst.

19. Komplementationszelllinie nach Anspruch 17 oder 18, welche ferner ein Konstrukt, welches ein virales gag/pol-Gen exprimiert, umfasst.

20. Komplementationszelllinie nach Anspruch 19, **dadurch gekennzeichnet, dass** sich das retrovirale gag/pol-Gen von MuLV oder FB 29 oder SIV ableitet.

21. Komplementationszelllinie nach einem der Ansprüche 17 bis 20, **dadurch gekennzeichnet, dass** die Zelllinie sich von der Linie 293 ableitet.

22. Verfahren zur Herstellung eines pseudotypisierten Viruspartikels nach Anspruch 16, welches umfasst:
(i) einen rekombinanten retroviralen Vektor in eine Komplementationszelle nach einem der Ansprüche 17 bis 21 einzuführen,
(ii) die Komplementationszelllinie unter geeigneten Bedingungen, um die Produktion des pseudotypisierten Viruspartikels zu ermöglichen, zu kultivieren und
(iii) das pseudotypisierte Viruspartikel aus der Zellkultur zu gewinnen.

23. Säugetierzelle, welche mit einem pseudotypisierten Viruspartikel nach Anspruch 16 oder mit einem pseudotypisierten Viruspartikel, welches durch ein Verfahren nach Anspruch 22 erhalten werden kann, infiziert ist.

24. Pharmazeutische Zusammensetzung, welche eine therapeutisch oder prophylaktisch wirksame Menge eines pseudotypisierten Viruspartikels nach Anspruch 16, oder welches durch ein Verfahren nach Anspruch 22 erhalten werden kann, oder einer Säugetierzelle nach Anspruch 23 umfasst.

25. Verwendung eines pseudotypisierten Viruspartikels nach Anspruch 16, oder welches durch ein Verfahren nach Anspruch 22 erhalten werden kann, oder einer Säugetierzelle nach Anspruch 23 zur Herstellung eines Arzneimittels, welches für die Behandlung einer genetisch-bedingten Erkrankung, einer Krebserkrankung oder einer viral induzierten Erkrankung bestimmt ist.

26. Verwendung eines Konstrukts, wie in einem der Ansprüche 1 bis 14 definiert, für die Expression eines Proteins für die Produktion von pseudotypisierten Viruspartikeln, wobei das Protein wenigstens ein modifiziertes Hüllprotein eines Foamy-Virus (FV) umfasst und die Modifikation zumindest eine Verkürzung an Rest 975 oder 981 des Proteins ist.

27. Verwendung eines Konstrukts nach Anspruch 26, **dadurch gekennzeichnet, dass** das Foamy-Virus das humane Foamy-Virus (HFV) ist.

## Revendications

1. Construction pour l'expression d'une protéine comprenant au moins une protéine modifiée d'enveloppe de virus spumeux (FV) pour la production de particules virales pseudotypées, la modification étant au moins une troncature au niveau du résidu 975 ou 981 de ladite protéine, **caractérisé en ce que** ladite protéine est une protéine de fusion comprenant en outre la totalité ou une partie d'une protéine d'enveloppe de non-FV.

2. Construction pour l'expression d'une protéine selon la revendication 1, **caractérisée en ce que** le virus spumeux est le virus spumeux humain (HFV).

3. Construction pour l'expression d'une protéine selon la revendication 1 ou 2, **caractérisée en ce que** la protéine d'enveloppe de non-FV est dérivée d'un virus choisi dans le groupe comprenant MuLV, MoMuLV, FB 29, HIV et SIV.

4. Construction pour l'expression d'une protéine selon la revendication 3, **caractérisée en ce que** ladite fusion est dans le domaine d'ancrage transmembranaire desdites protéines d'enveloppe de FV et de non-FV.

5. Construction pour l'expression d'une protéine selon la revendication 4, **caractérisée en ce que** la protéine comprend le domaine extracellulaire et la partie 5' du domaine d'ancrage transmembranaire de la protéine d'enveloppe de HFV et la partie 3' du domaine d'ancrage transmembranaire et le domaine cytoplasmique de la protéine d'enveloppe de non-FV, en particulier de la protéine d'enveloppe de SIV.

6. Construction pour l'expression d'une protéine selon la revendication 3, **caractérisée en ce que** ladite fusion est dans le site de coupure desdites protéines d'enveloppe de FV et de non-FV.

7. Construction pour l'expression d'une protéine selon la revendication 6, **caractérisée en ce que** la protéine comprend le domaine SU et la totalité ou une partie du site de coupure de la protéine d'enveloppe de HFV et la totalité ou une partie du site de coupure et du domaine TM, comprenant le domaine d'ancrage transmembranaire et le domaine cytoplasmique, de la protéine d'enveloppe de non-FV, en particulier de la protéine d'enveloppe de SIV.

8. Construction pour l'expression d'une protéine selon la revendication 3, **caractérisée en ce que** ladite fusion est à la jonction entre le domaine d'ancrage transmembranaire et le domaine cytoplasmique ou à l'intérieur des domaines cytoplasmiques desdites protéines d'enveloppe de FV et de non-FV.

9. Construction pour l'expression d'une protéine selon la revendication 8, **caractérisée en ce que** la protéine comprend le domaine extracellulaire, le domaine d'ancrage transmembranaire et la totalité ou une partie du domaine cytoplasmique de la protéine d'enveloppe de HFVe et la totalité ou une partie du domaine cytoplasmique de la protéine d'enveloppe de non-FV, en particulier de la protéine d'enveloppe de SIV.

10. Construction pour l'expression d'une protéine selon l'une quelconque des revendications 1 à 9, **caractérisée en ce que** la protéine consiste en la protéine d'enveloppe de HFV dont la totalité ou une partie du domaine cytoplasmique est remplacée par la totalité ou une partie d'une protéine d'enveloppe virale de non-FV, et de préférence d'une protéine d'enveloppe rétrovirale de MuLV.

11. Construction pour l'expression d'une protéine selon l'une quelconque des revendications 1 à 10, **caractérisée en ce que** la protéine consiste en la fusion de la totalité ou d'une partie d'un domaine cytoplasmique de MuLV avec une protéine modifiée d'enveloppe de HFV.

12. Construction pour l'expression d'une protéine selon les revendications 10 et 11, **caractérisée en ce que** le domaine cytoplasmique de MuLV est maturé ou non maturé.

13. Construction pour l'expression d'une protéine selon l'une quelconque des revendications 1 à 12, **caractérisée en ce que** la protéine comprend une protéine modifiée d'enveloppe de HFV tronquée du résidu 981 qui est fusionné à un résidu arginine et le domaine cytoplasmique non maturé de MuLV s'étendant du résidu 634 au résidu 665 de la protéine d'enveloppe de MuLV de type sauvage.

14. Construction pour l'expression d'une protéine selon l'une quelconque des revendications 1 à 13, **caractérisée en ce que** le produit de construction comprend une mutation du site d'épissage donneur et/ou du site d'épissage accepteur présent(s) à l'état naturel dans la séquence codant la protéine d'enveloppe de FV.

15. Protéine telle qu'exprimée par une construction selon l'une quelconque des revendications 1 à 14.

16. Particule virale pseudotypée comprenant une protéine selon la revendication 15.

17. Lignée cellulaire de complémentation comprenant une construction selon l'une quelconque des revendications 1 à 14.

18. Lignée cellulaire de complémentation selon la revendication 17, comprenant une construction selon la revendication 9 ou 13.

19. Lignée cellulaire de complémentation selon la revendication 17 ou 18, comprenant en outre une construction exprimant un gène gag/pol viral.

20. Lignée cellulaire de complémentation selon la revendication 19, **caractérisée en ce que** le gène gag/pol rétroviral est issu de MuLV ou FB 29 ou SIV.

21. Lignée cellulaire de complémentation selon l'une quelconque des revendications 17 à 20, **caractérisée en ce que** la lignée cellulaire dérive de la lignée 293.

22. Procédé pour la production d'une particule virale pseudotypée selon la revendication 16, comprenant :
(i) l'introduction d'un vecteur rétroviral recombinant dans une cellule de complémentation selon l'une quelconque des revendications 17 à 21,
(ii) la culture de ladite lignée cellulaire de complémentation dans des conditions appropriées, pour permettre la production de ladite particule virale pseudotypée, et
(iii) la récupération de ladite particule virale pseudotypée à partir de la culture de cellules.

23. Cellule de mammifère infectée par une particule virale pseudotypée selon la revendication 16 ou par une particule virale pseudotypée pouvant être obtenue par un procédé selon la revendication 22.

24. Composition pharmaceutique comprenant une quantité thérapeutiquement ou prophylactiquement efficace d'une particule virale pseudotypée selon la revendication 16 ou pouvant être obtenue par un procédé selon la revendication 22 ou d'une cellule de mammifère selon la revendication 23.

25. Utilisation d'une particule virale pseudotypée selon la revendication 16 ou pouvant être obtenue par un procédé selon la revendication 22 ou d'une cellule de mammifère selon la revendication 23, pour la fabrication d'un médicament destiné au traitement d'un trouble génétique, d'un cancer ou d'une maladie d'origine virale.

26. Utilisation d'un produit de construction tel que défini dans l'une quelconque des revendications 1 à 14, pour l'expression d'une protéine pour la production de particules virales pseudotypées, ladite protéine comprenant au moins une protéine modifiée d'enveloppe de virus spumeux (FV) et ladite modification étant au moins une troncature au niveau du résidu 975 ou 981 de ladite protéine.

27. Utilisation d'un produit de construction selon la revendication 26, **caractérisée en ce que** le virus spumeux est le virus spumeux humain (HFV).


### SEQUENCE LISTING

(1) GENERAL INFORMATION:
   (i) APPLICANT:
      (A) NAME: TRANSGENE S.A.
      (B) STREET: 11 rue de Molsheim
      (C) CITY: Strasbourg
      (E) COUNTRY: France
      (F) POSTAL CODE (ZIP): 67000
      (G) TELEPHONE: 03 88 27 91 00
      (H) TELEFAX: 03 88 27 91 41
   (ii) TITLE OF INVENTION: expression of a foamy virus envelop protein
   (iii) NUMBER OF SEQUENCES: 6
   (iv) COMPUTER READABLE FORM:
      (A) MEDIUM TYPE: Tape
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (C) INDIVIDUAL ISOLATE: oligonucleotide de synthese oTG11854
   (xi) SEQUENCE DESCRIPTION: SEQ ID NO: 1:
   TGGAACTGCC TTTAGTCTCT TGGGATACTT AAAGCCTATC CTAATAGGAG TAGGAGTAAT    60
   ACTGTTAAGA                                                           70
(2) INFORMATION FOR SEQ ID NO: 2:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: YES
   (vi) ORIGINAL SOURCE:
      (C) INDIVIDUAL ISOLATE: oligonucleotide de synthese oTG11855
   (xi) SEQUENCE DESCRIPTION: SEQ ID NO: 2:
   GCCCTCGAGG AATTCTCACA AGAGCGTGAG CTCAA 35
(2) INFORMATION FOR SEQ ID NO: 3:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: YES
   (vi) ORIGINAL SOURCE:
      (C) INDIVIDUAL ISOLATE: oligonucleotide de synthese oTG11856
   (xi) SEQUENCE DESCRIPTION: SEQ ID NO: 3:
   GCCCTCGAGG AATTCCTACT GGAAATAAGA GGGTG 35
(2) INFORMATION FOR SEQ ID NO: 4:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (C) INDIVIDUAL ISOLATE: oligonucleotide de synthese oTG11857
   (xi) SEQUENCE DESCRIPTION: SEQ ID NO: 4:
   CTCCTTCCTA TCCCAATGTT ACTAGGGAAC ATTATACTTC CTGTAATAAT AGAAATAAAA 60
   GAGGGGTCTT 70
(2) INFORMATION FOR SEQ ID NO: 5:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (C) INDIVIDUAL ISOLATE: oligonucleotide de synthese oTG11858
   (xi) SEQUENCE DESCRIPTION: SEQ ID NO: 5:
   TAGGGGTCAT TCTCTTGGTT ATTCTTATAT TTAAAATTGT ATCCTGGATT AAGTTAAGGC 60
   AGGGGTATAG 70
(2) INFORMATION FOR SEQ ID NO: 6:
   (i) SEQUENCE CHARACTERISTICS:
      (A) LENGTH: 35 base pairs.
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: YES
   (vi) ORIGINAL SOURCE:
      (C) INDIVIDUAL ISOLATE: oligonucleotide de synthese oTG11866
   (xi) SEQUENCE DESCRIPTION: SEQ ID NO: 6:
   GCCCTCGAGG AATTCCTATT GGAAATAAGA GGGTG 35